# EUROPÄISCHE PATENTANMELDUNG

(11) **EP 4 417 167 A1**
(43) Veröffentlichungstag der Anmeldung: **21.08.2024**
(21) Anmeldenummer: 24157502.6
(22) Anmeldetag: 14.02.2024
(51) Int. Cl.: A61F 2/91

(54) **MEDIZINISCHE VORRICHTUNG UND BEHANDLUNGSSYSTEM**

(30) Priorität: 20.02.2023 DE 102023104170
(71) Anmelder: Acandis GmbH, 75177 Pforzheim (DE)
(72) Erfinder: LANGE, Alexander, 76135 Karlsruhe (DE)
(74) Vertreter: Meissner Bolte Partnerschaft mbB

(57) **Zusammenfassung**

Die Erfindung betrifft eine Medizinische Vorrichtung zur Einfuhr in ein Körperorgan mit einer komprimierbaren und expandierbaren Gitterstruktur (10) aus Stegen (11), die durch Stegverbinder (12) miteinander verbunden sind und geschlossene Zellen (13) der Gitterstruktur (10) begrenzen, wobei jeweils zwei Stege (11a, 11b) wenigstens einer Zelle (13) gegenüber angeordnet sind und ein erstes Stegpaar (14a) und ein zweites Stegpaar (14b) bilden, wobei eines der beiden Stegpaare (14a, 14b) durch mindestens einen Verbindungssteg (15) miteinander verbunden ist, der sich in die Zelle (13) erstreckt und diese überbrückt.

## Beschreibung

Die Erfindung betrifft eine medizinische Vorrichtung zur Einfuhr in ein Körperorgan mit einer komprimierbaren und expandierbaren Gitterstruktur aus Stegen. Die Stege sind durch Stegverbinder miteinander verbunden und begrenzen geschlossene Zellen der Gitterstruktur. Jeweils zwei Stege wenigstens einer Zelle sind gegenüber angeordnet und bilden ein erstes Stegpaar und ein zweites Stegpaar.

Eine derartige medizinische Vorrichtung ist beispielsweise aus EP 1 903 999 B1 bekannt, die einen Stent beschreibt, der ein geschlossenzelliges Design aufweist (Closed-Cell-Design). Bei derartigen Stents besteht das Problem, dass diese gerade in stark gekrümmten Gefäßen eine nicht immer ausreichende Biegeflexibilität aufweisen, um gut an der Gefäßwand anzuliegen (Wandapposition). Die im eingangs genannten Stand der Technik getroffenen Maßnahmen zur Verbesserung der Biegeflexibilität sind aufwendig.

Der Erfindung liegt die Aufgabe zugrunde, die Biegeflexibilität der Gitterstruktur einer medizinischen Vorrichtung auf einfache Art und Weise zu verbessern, ohne das geschlossenzellige Design aufzugeben.

Erfindungsgemäß wird diese Aufgabe durch eine medizinische Vorrichtung mit den Merkmalen des Anspruchs 1 gelöst.

Der Erfindung liegt ferner die Aufgabe zugrunde, ein Behandlungssystem mit einer derartigen medizinischen Vorrichtung anzugeben. Diese Aufgabe wird erfindungsgemäß durch den Gegenstand des Anspruchs 11 gelöst.

Konkret wird die Aufgabe durch eine medizinische Vorrichtung zur Einfuhr in ein Körperorgan mit einer komprimierbaren und expandierbar Gitterstruktur aus Stegen gelöst. Die Stege sind durch Stegverbinder miteinander verbunden und begrenzen geschlossene Zellen der Gitterstruktur. Jeweils zwei Stege wenigstens einer Zelle sind gegenüber angeordnet und bilden ein erstes Stegpaar und ein zweites Stegpaar. Eines der beiden Stegpaare ist durch mindestens einen Verbindungssteg miteinander verbunden. Der Verbindungssteg erstreckt sich in die Zelle und überbrückt diese.

Die Erfindung hat verschiedene Vorteile.

Der Verbindungssteg bietet die konstruktive Voraussetzung dafür, die Gitterstruktur mit geschlossenen Zellen hinsichtlich der Biegeflexibilität zu optimieren, ohne dass dabei die Stützkraft bzw. Radialkraft der Gitterstruktur beeinträchtigt oder zumindest wesentlich beeinträchtigt wird. Die Erfindung ermöglicht eine sehr gute Gefäßapposition in gewundenen Gefäßen bei hoher Stützkraft bzw. Radialkraft. Risiken, die die bei schlechter Wandapposition auftreten können, wie Migration oder negative hämodynamische Effekte, sinken dadurch beträchtlich. Dazu ist wenigstens ein Verbindungssteg vorgesehen, der eines der beiden Stegpaare miteinander verbindet, sich in die Zelle hinein erstreckt und diese überbrückt. Durch den zusätzlichen Verbindungssteg wird eine erhöhte Stützkraft erzielt, so dass andere Eigenschaften oder Merkmale der Gitterstruktur zur Verbesserung der Biegeflexibilität verändert werden können, wie beispielsweise die Anzahl der Zellen auf dem Umfang, ohne dass das Risiko der Migration steigt.

Das geschlossenzellige Design (Closed-Cell-Design) hat den Vorteil der guten Wiedereinziehbarkeit zur Repositionierung der Vorrichtung im Gefäß (Resheathability).

Der zusätzliche Verbindungssteg ist einfach, beispielsweise durch Laserschneiden, herstellbar. Die gute Repositionierbarkeit der Gitterstruktur wird nicht beeinträchtigt.

Ein weiterer Vorteil der Erfindung besteht darin, dass die Grundform der Gitterstruktur beibehalten werden kann. Die Form der Zelle kann als geschlossene Zelle beibehalten werden. Der zusätzliche Verbindungssteg wird im Design der Zelle ergänzt. Das Überbrücken der Zelle durch den Verbindungssteg ist so zu verstehen, dass das Design der Grundzelle zusätzlich den wenigstens einen Verbindungssteg umfasst, der sich über die gesamte Grundzelle zwischen zwei gegenüber angeordneten Stegen erstreckt. Der Verbindungssteg überbrückt bzw. überspannt die gesamte Zelle und verbindet die beiden gegenüberliegenden Stege eines Stegpaares.

Im Allgemeinen gilt, dass die gegenüberliegenden Stege eines (ersten) Stegpaares nicht direkt miteinander verbunden sind, sondern jeweils mit den gegenüberliegenden Stegen eines weiteren (zweiten) Stegpaares sowie mit dem Verbindungssteg.

Das Stegpaar bildet zusammen mit dem weiteren Stegpaar die Begrenzung der Zelle. Dabei ist nicht ausgeschlossen, dass die Zelle durch weitere Stege begrenzt ist. Es ist auch möglich, dass mehr als ein Verbindungssteg in die Zelle bzw. in die jeweilige Zelle hineinragt und diese überbrückt.

Die gegenüberliegenden Stege der Stegpaare können gerade und/oder gekrümmt sein. Zum Grunddesign der Zelle wird auf die Patente der Anmelderin verwiesen.

EP 2 667 831 B1 schützt das grundlegende Prinzip der Rotation bei Expansion über paarweise gegenüberliegende Stege mit unterschiedlicher Biegesteifigkeit. DE 10 2013 104 550 B4 beschreibt ein Zellendesign und die Form der Stegverbinder, wobei die Stegverbinder X-förmig ausgebildet sind. Mittels ungleicher Stegbreiten jeweils benachbart liegender Stege wird eine Torsion der Stentstruktur beim Expandieren erzielt. DE 10 2013 107 258 B4 beschreibt zusätzlich einen kompletten Stentaufbau mit Rand- und Übergangszellen, Querschnittsform mit Flaring sowie Randzellen mit Markern.

Die Erfindung ist auf diese geschützten Technologien anwendbar und wird im Zusammenhang mit den in den eigenen Patenten der Anmelderin beschriebenen Zelldesigns offenbart und beansprucht. Dies schließt nicht aus, dass die Erfindung auf weitere, hier nicht beschriebene Zelldesigns anwendbar ist.

Das erfindungsgemäße Prinzip gilt für wenigstens eine Zelle der Gitterstruktur. Das Prinzip kann auf mehrere Zellen der Gitterstruktur, insbesondere auf mehrere auf dem Umfang der Gitterstruktur verteilt angeordnete Zellen ausgeweitet werden. Die vorstehenden im Zusammenhang mit wenigstens einer Zelle beschriebenen und beanspruchten Merkmale werden auch im Zusammenhang mit mehreren Zellen, insbesondere mit mehreren auf einem Umfangssegment verteilt angeordneten Zellen beschrieben und beansprucht.

Die erfindungsgemäße Vorrichtung eignet sich bspw. als temporäres oder permanentes Gefäßimplantat. Sie kann etwa ein Stent oder ein Thrombektomiedevice sein. Andere Anwendungen sind denkbar.

Vorteilhafterweise ist die Gitterstruktur im Wesentlichen röhrchenförmig.

Die Gitterstruktur kann selbstexpandierend sein.

Bevorzugte Ausführungsformen der Erfindung sind in den Unteransprüchen angegeben.

Vorzugsweise ist die Zelle im Wesentlichen rautenförmig. Dies hat den Vorteil, dass der Verbindungssteg im Zusammenhang mit einer bewährten geschlossenzelligen Form verwendet wird, die sich mit Blick auf die Biegeflexibilität gut optimieren lässt. Rautenförmig bedeutet nicht im streng geometrischen Sinn, dass die Stege gerade sein müssen. Es genügt, wenn die Grundform der Zelle rautenförmig ist. Die jeweiligen Stegpaare können jeweils gerade und/oder gekrümmte paarweise gegenüberliegende Stege aufweisen.

Bei einer bevorzugten Ausführungsform sind mehrere Zellen auf wenigstens einem Umfangssegment angeordnet, bei denen jeweils eines der beiden Stegpaare durch mindestens einen Verbindungssteg miteinander verbunden ist, der sich in die Zelle erstreckt und diese überbrückt. Das Übertragen des Prinzips einer Zelle auf mehrere Zellen eines Umfangssegments bewirkt, dass die Stützkraft durch die zusätzlichen Verbindungsstege der jeweiligen Zellen auf dem gesamten Umfang im Bereich des Umfangssegments erhöht wird. Die Anzahl der Zellen auf dem Umfangssegment ist nicht eingeschränkt. Es sind mehrere Umfangssegmente in axialer Richtung möglich, die jeweils entsprechende Zellen mit jeweils wenigstens einem Verbindungssteg aufweisen.

Das Umfangssegment mit den Zellen mit Verbindungsstegen kann als erstes Umfangssegment bezeichnet werden.

Bei einer bevorzugten Ausführungsform sind die Stegverbinder des ersten Umfangssegments auf dem Umfang um einen Winkel versetzt, der kleiner oder gleich 180° ist. Dies hat den Vorteil, dass die Gitterstruktur - im Fall einer röhrchenförmigen Gitterstruktur - beim Crimpen in einem Katheter auf einen besonders kleinen Durchmesser komprimiert werden kann. Die Stegverbinder werden durch die versetzte Anordnung besonders eng angeordnet, wodurch kleine Durchmesser der Gitterstruktur im gecrimpten Zustand erzielt werden.

Beispielsweise sind drei auf dem Umfangssegment, insbesondere ersten Umfangssegment angeordnete Zellen so ausgebildet, dass die Stegverbinder - im Querschnitt der Gitterstruktur gesehen - um 120° versetzt angeordnet sind. Die Erfindung bzw. das vorliegende Ausführungsbeispiel ist nicht auf drei Zellen pro Umfangssegment eingeschränkt. Die versetzte Anordnung der Stegverbinder bezogen auf den Querschnitt der Gitterstruktur kann auch mit einer anderen Anzahl von Zellen erreicht werden.

Als Querschnitt der Gitterstruktur wird ein Schnitt angesehen, der orthogonal zur Längsachse der Gitterstruktur verläuft.

Bei einer besonders bevorzugten Ausführungsform der Erfindung überbrückt der Verbindungssteg die Zelle diagonal. Dadurch wird eine besonders hohe Stützkraft erreicht.

Die diagonale Anordnung des Verbindungssteges wird im Zusammenhang mit wenigstens einer Zelle, insbesondere im Zusammenhang mit allen Zellen des ersten Umfangssegments offenbart und beansprucht. Es können mehrere Umfangssegmente in axialer Richtung vorgesehen sein, die entsprechend dem ersten Umfangssegment mit diagonalen Verbindungsstegen ausgestattet sind.

Dabei bedeutet die diagonale Anordnung, dass der Verbindungssteg bezogen auf zumindest eine Achse der Zelle, insbesondere auf beide Achsen der Zelle schräg verläuft bzw. allgemein von der Lage der Achse bzw. der Achsen abweicht, d. h. der Verbindungssteg schneidet die Achse bzw. die Achsen. Bspw. verläuft bei einer rautenförmigen Zelle die Längsachse der Zelle zwischen den beiden Spitzen der rautenförmigen Zelle, die den größeren Abstand haben. Die Hochachse ist kürzer als die Längsachse. Die Lage der Achsen kann sich in Abhängigkeit vom Expansions- bzw. Kompressionszustand bezogen auf die Längsachse der gesamten Vorrichtung ändern.

Die diagonale Anordnung bedeutet, dass der Verbindungssteg nur eines der beiden Stegpaare mit einander verbindet. Das andere Stegpaar ist mit Blick auf den Verbindungssteg frei.

Der diagonal angeordnete Verbindungssteg kann gerade oder gekrümmt ausgebildet sein. Der gekrümmte Verbindungssteg ist vorteilhafterweise S-förmig ausgebildet. Andere Formen des Verbindungssteges sind möglich.

Vorzugsweise ist der Verbindungssteg mit dem Stegpaar jeweils durch einen flexiblen, insbesondere einen Z-förmigem Stegverbinder verbunden. Der flexible Stegverbinder, insbesondere der Z-förmige Stegverbinder weist eine größere Flexibilität auf als X-förmige Stegverbinder. Dies hat den Vorteil, dass die Biegeflexibilität weiter verbessert wird.

Der flexible Stegverbinder erlaubt eine Relativbewegung zwischen dem Verbindungssteg und dem Stegpaar, wenn die Gitterstruktur bewegt, bspw. gekrümmt oder expandiert/komprimiert wird. Der flexible Stegverbinder kann bspw. Z-förmig sein.

Bei einer weiteren vorzugsweisen Ausführungsform sind die Verbindungsstege einer ersten Zelle und die Verbindungsstege einer in Umfangsrichtung benachbarten zweiten Zelle versetzt zueinander angeordnet. Ein Beispiel dafür, wie die versetzte Anordnung der Verbindungsstege zweier benachbarter Zellen erreicht werden kann, ist die Verbindung des jeweiligen Verbindungssteges mit dem zugehörigen Stegpaar durch einen Z-förmigem Stegverbinder.

Andere Möglichkeiten der Anbindung der Verbindungsstege sind denkbar, die bewirken, dass die Verbindungsstege zweier in Umfangsrichtung benachbarter Zellen versetzt zueinander angeordnet sind.

Die Z-förmige Anbindung der Verbindungsstege bewirkt, dass die diagonalen Verbindungsstege zweier benachbarter Zellen nicht fluchten, sondern dass deren Verbindungsstellen mit der Zelle, insbesondere der rautenförmigen Zelle so zueinander versetzt sind, dass sich im geladenen Zustand, also im komprimierten Zustand im Katheter, Teile dieser benachbarten diagonal angeordneten Verbindungsstege überlappen.

Dabei kann der Versatz zwischen den Einmündungsstellen bzw. Verbindungsstellen der diagonal angeordneten Verbindungsstege an den jeweiligen Stegpaaren, d. h. die Länge der Überlappung, zwischen dem 0,1- und dem 0,9-fachen der Steglänge der Zelle, insbesondere der Rautenzelle betragen. Der Versatz kann zwischen dem 0,2- und 0,8-fachen, insbesondere zwischen dem 0,3- und 0,7 fachen der Steglänge der Zelle, insbesondere der Rautenzelle betragen.

Vorzugsweise ist die Stegbreite des Verbindungssteges im Bereich seiner Enden kleiner als die Stegbreite in einem Bereich, der sich zwischen den Enden des Verbindungssteges erstreckt. Dadurch wird die Wandadaption weiter verbessert, da der diagonale Steg der Zelle, die an der Innenseite einer Gefäßbiegung liegt und daher gestaucht wird, dann weniger dazu tendiert, sich durch die Stauchung ins Gefäßinnere zu biegen.

Weiter vorzugsweise ist die Stegbreite der Stege des ersten und/oder zweiten Stegpaares in einem Bereich nahe der X-förmigen Stegverbinder kleiner als die Stegbreite in einem Bereich nahe der Z-förmigen Stegverbinder. Die Stege des ersten und/oder zweiten Stegpaares sind dabei vorzugsweise in Richtung der X-förmigen Stegverbinder verjüngt ausgebildet. Dadurch wird eine gute Biegeflexibilität der Stege bzw. der Gitterstruktur erreicht.

Das Verhältnis der Stegbreite der Stege im Bereich nahe der X-förmigen Stegverbinder zur Stegbreite nahe der Z-förmigen Stegverbinder beträgt vorzugsweise zwischen 0,5 und 1,0, insbesondere ca. 0,75. Das Verhältnis der Stegbreite im Bereich der Enden des Verbindungsstegs zur Stegbreite im Bereich zwischen den Enden des Verbindungsstegs beträgt zwischen 0,7 und 1,0, insbesondere ca. 0,95. Die Stege des ersten und/oder zweiten Stegpaares im Bereich nahe der X-förmigen Stegverbinder sind folglich vorzugsweise stärker verjüngt ausgebildet als die Enden des Verbindungsstegs.

Bei einer konkreten Ausführungsform weist das Umfangssegment wenigstens 2, insbesondere 3 Zellen auf. Dadurch wird die Biegeflexibilität gegenüber einer Ausführungsform mit mehr Zellen auf dem Umfang verbessert. Vorzugsweise weist die Gitterstruktur maximal 5 Zellen pro Umfangssegment auf. Die Randbereiche können mehr als 5 Zellen pro Umfangssegment, bspw. 6 Zellen aufweisen. Andere Designs sind möglich.

Die Anzahl der Zellen auf dem ersten Umfangssegment ist vorteilhafterweise geringer als die Anzahl der Zellen auf wenigstens einem zweiten Umfangssegment, das in axialer Richtung der Gitterstruktur proximal und/oder distal entfernt vom ersten Umfangssegment angeordnet ist. Bei dieser Ausführungsform sind mehrere Umfangssegmente in axialer Richtung der Gitterstruktur vorgesehen, wobei die relativ zum ersten Umfangssegment axial weiter außen angeordneten Umfangssegmente bzw. ein weiter außen angeordnetes Umfangssegment mehr Zellen auf dem Umfang aufweist als das erste Umfangssegment.

Diese Ausführungsform hat den Vorteil, dass die in axialer Richtung der Gitterstruktur weiter innen angeordneten Zellen, die auch als Mittelzellen oder innere Zellen bezeichnet werden, gut mit Blick auf die Biegeflexibilität optimiert werden können, weil die Mittelzellen jeweils Verbindungsstege aufweisen, sodass in diesem Bereich die Flexibilität besonders hoch ist, die Stützwirkung aber nicht sonderlich reduziert wird, da die Radialkraft gleichmäßig auf die Gefäßoberfläche verteilt wird.

Die relativ zu dem ersten Umfangssegment bzw. zu den Mittelzellen weiter außen angeordneten Außenzellen bzw. äußeren Zellen oder Randzellen weisen im Vergleich zu den Mittelzellen keine Verbindungsstege auf. Die Außenzellen können andere Funktionen oder Eigenschaften aufweisen, wie beispielsweise Flaring oder Röntgensichtbarkeit durch Röntgenmarker.

Auf einen Stent oder ein Thrombektomie-Device übertragen hat diese Ausführungsform den Vorteil, dass im mittleren Bereich der Gitterstruktur, dort wo besonders starke Krümmungen auftreten, die Zellen der Gitterstruktur durch die zusätzlichen Verbindungsstege verstärkt sind, sodass dort die Biegeflexibilität optimiert werden kann. Dies wird bei dieser erfindungsgemäßen Ausführungsform dadurch erreicht, dass die Anzahl der rautenförmigen Zellen auf dem ersten Umfangssegment geringer ist als die Anzahl der Zellen auf wenigstens einem zweiten Umfangssegment, das in axialer Richtung der Gitterstruktur proximal und/oder distal entfernt vom ersten Umfangssegment angeordnet ist.

Die mit einer geringen Anzahl von Zellen üblicherweise einhergehende schlechtere Verteilung der Stützwirkung wird durch die Verstärkung dieser Zellen durch die zusätzlichen Verbindungsstege kompensiert, sodass in diesem Bereich der Gitterstruktur eine sehr gute Biegeflexibilität bei gleichzeitig gut verteilter Stützkraft erreicht wird. Die Verteilung der Stützkraft auf eine größere Anzahl von Stegen im Vergleich zu einem Umfangsegment mit gleicher Zellenanzahl ohne Verbindungsstege bedeutet, dass die Stützkraft nicht auf wenige Kontaktlinien konzentriert ist, bzw. anders gesagt, durch mehrere Kontaktlinien übertragen wird. Die Verbindungsstege tragen auch zu einer Erhöhung der Stützkraft bei.

Das Verhältnis der Anzahl der Mittelzellen pro Umfangssegment zur Anzahl der Außenzellen pro Umfangssegment kann 1:1, 1:2, 1:3 oder 1:4 betragen. Andere Verhältnisse sind bei dieser Ausführungsform möglich, solange weniger Mittelzellen pro Umfangssegment als Außenzellen pro Umfangssegment vorgesehen sind.

Das erfindungsgemäße Grundprinzip kann auch bei einer Ausführungsform verwirklicht sein, die eine konstante Zellenanzahl pro Umfangssegment entlang der gesamten Länge der Gitterstruktur aufweist.

Dabei können bei gleicher Zellanzahl das Zelldesign der Außenzellen und das Zelldesign der Mittelzellen unterschiedlich sein. Andere Unterschiede sind möglich.

Die Mittelzellen können auch als mittlere Zellen oder innere Zellen und die Au-ßenzellen auch als äußere Zellen bezeichnet sein. Es kommt lediglich auf die relative Anordnung der Zellen zueinander an.

Das erfindungsgemäße Grundprinzip ist ebenfalls für eine medizinische Vorrichtung anwendbar, die eine Membran aufweist, wobei die Membran die Zellen der Gitterstruktur zumindest teilweise abdeckt. Die Membran kann als elektrogesponnenen Membran ausgebildet sind. In diesem Fall ist es bevorzugt, wenn 9 Zellen pro Umfangsegment vorgesehen sind, da auf diese Weise das Einströmen von Blut zwischen der Gefäßwand und der Membran weitgehend verhindert bzw. reduziert werden kann.

Das Behandlungssystem weist eine erfindungsgemäße medizinische Vorrichtung bzw. eine medizinische Vorrichtung nach einer der vorstehend genannten Ausführungsformen sowie einen Katheter auf, in dem ein Führungselement mit einem axialen Ende der Gitterstruktur verbunden ist.

Die Erfindung wird nachstehend anhand von Ausführungsbeispielen unter Bezug auf die beigefügten schematischen Zeichnungen mit weiteren Einzelheiten näher beschrieben. In diesen zeigen
- Fig. 1: eine Abwicklung der Gitterstruktur eines erfindungsgemäßen Ausführungsbeispiels der medizinischen Vorrichtung, insbesondere eines Stents, mit 3 Zellen pro Umfangssegment
- Fig. 2: einen vergrößerten Ausschnitt der Gitterstruktur gemäß Fig. 1 im Bereich einer mittleren Zelle mit einem Verbindungssteg, wobei die Stegbreiten S1-S4 eingezeichnet sind;
- Fig. 3: eine Abwicklung der Gitterstruktur eines erfindungsgemäßen Ausführungsbeispiels der medizinischen Vorrichtung, insbesondere eines Stents, mit 2 Zellen pro Umfangssegment;
- Fig. 4: eine schematische Gegenüberstellung einer Gitterstruktur mit einer großen Anzahl von Zellen pro Umfangssegment (links - Vergleichsbeispiel) und einer im Vergleich dazu geringeren Anzahl von Zellen pro Umfangssegment (rechts - erfindungsgemäßes Beispiel) in stark gekrümmtem Zustand;
- Fig. 5: einen Querschnitt durch eine Gitterstruktur mit 6 Zellen pro Umfangssegment und der Anordnung der Stegverbinder auf dem Umfang der Gitterstruktur (Vergleichsbeispiel);
- Fig. 6: einen Querschnitt durch eine Gitterstruktur mit 3 Zellen pro Umfangssegment und der Anordnung der Verbinder auf dem Umfang der Gitterstruktur (erfindungsgemäßes Beispiel);
- Fig. 7: eine Abwicklung der Gitterstruktur eines weiteren erfindungsgemäßen Ausführungsbeispiels der medizinischen Vorrichtung, insbesondere eines Stents, mit 3 Zellen pro Umfangssegment; und
- Fig. 8: einen vergrößerten Ausschnitt der Gitterstruktur gemäß Fig. 7 im Bereich einer mittleren Zelle mit einem Verbindungssteg, wobei die Stegbreiten S1-S4 eingezeichnet sind.

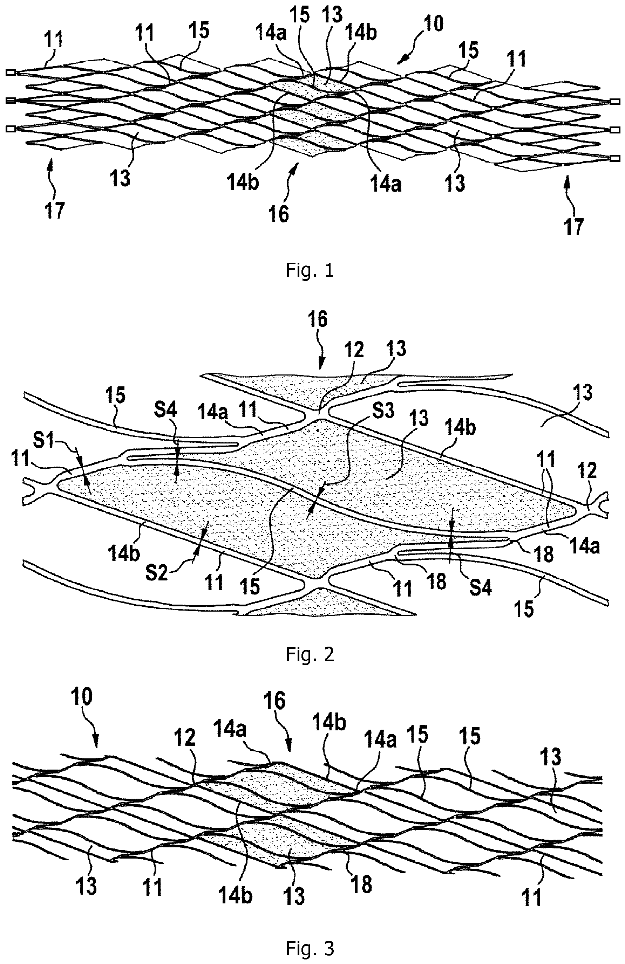

Figuren 1, 2, 3, 7 und 8 zeigen erfindungsgemäße Ausführungsbeispiele einer medizinischen Vorrichtung zur Einfuhr in ein Körperorgan. Dabei handelt es sich um die Anwendung der Vorrichtung als Stent bzw. allgemein als temporäres oder permanentes Gefäßimplantat. Andere Anwendungen sind denkbar, beispielsweise als Thrombektomie-Device. Die Vorrichtung weist eine komprimierbare und expandierbare Gitterstruktur 10 auf, die aus Stegen 11 gebildet ist. Die Gitterstruktur 10 kann beispielsweise durch Laserschneiden hergestellt werden. Aus Gründen der besseren Übersichtlichkeit ist die Gitterstruktur in Figuren 1, 2 und 3 im abgewickelten Zustand gezeigt.

Im Gebrauch ist die Gitterstruktur 10 röhrchenförmig.

Die Stege 11 sind durch Stegverbinder 12, die X-förmig ausgebildet sind, miteinander verbunden. Die Stege 11 begrenzen jeweils eine geschlossene Zelle 13 der Gitterstruktur 10. Im hier gezeigten Ausführungsbeispiel ist die Zelle 13 rautenförmig. In den Figuren ist gut zu erkennen, dass dabei keine streng geometrische Rautenform gefordert ist. Die Seiten der Raute können teilweise gekrümmt und teilweise gerade sein. Die Grundform der Zelle 13 ist rautenförmig.

Die Zelle 13 ist von 4 Stegen 11 begrenzt, die jeweils paarweise gegenüber angeordnet sind und zwei Stegpaare 14a, 14b bilden. Die gegenüberliegenden Stege 11 des ersten Stegpaares 14a sind nicht direkt miteinander verbunden, sondern ergänzen sich mit den Stegen 11 des zweiten Stegpaares 14b zur rautenförmigen Grundform der Zelle 13.

Im Beispiel gemäß Figuren 1, 2 sind die Stege 11 des ersten Stegpaares 14a gekrümmt. Die Stege 11 des zweiten Stegpaares 14b sind gerade. Andere Grundformen der Zelle 13 sind möglich, solange die Zelle 13 geschlossen ist, wie in den Figuren 1, 2 dargestellt.

Die Stegbreite der Stege 11 des ersten Stegpaares 14a ist größer als die Stegbreite der Stege 11 zweiten Stegpaares 14b. Dadurch haben die Stege 11 des ersten Stegpaares 14a eine höhere Biegefestigkeit als die Stege 11 des zweiten Stegpaares 14b. Die dadurch bei der Expansion und Komprimierung der Gitterstruktur 10 bewirkte Rotation der Zelle 13 führt zu einer hervorragenden Biegeflexibilität der gesamten Gitterstruktur 10, die die Gitterstruktur von anderen Closed-Cell-Designs maßgeblich unterscheidet. Der Mechanismus ist in den Patenten der Anmelderin EP 2 667 831, DE 10 2013 104 550 B4 und DE 10 2013 107 258 B4 im Detail beschrieben und geschützt und wird an dieser Stelle nicht weiter vertieft.

Allgemein gilt, dass die im Zusammenhang mit einer Zelle 13 der Gitterstruktur 10 beschriebenen Merkmale und Funktionen auch für weitere Zellen 13 der Gitterstruktur 10 offenbart und beansprucht werden. Damit können Umfangssegmente 16 und aus mehreren Umfangssegmenten 16 bestehende axiale Abschnitte der Gitterstruktur entsprechende Zellen 13 aufweisen. Die Randzellen der Gitterstruktur weisen üblicherweise ein anderes Design auf, da dort andere Funktionen, wie beispielsweise Flaring zu erfüllen sind.

Die Gitterstruktur 10 weist insgesamt eine geschlossenzelliges Design auf. Alle Zellen 13 der Gitterstruktur 10 sind geschlossen. Andere Ausführungen der Gitterstruktur 10 sind möglich.

Wie in den Figuren 1, 2 gut zu erkennen, ist eines der beiden Stegpaare 14a, 14b, konkret das erste Stegpaar 14a, durch einen Verbindungssteg 15 miteinander verbunden. Der Verbindungssteg 15 erstreckt sich in die Zelle 13 hinein und überbrückt die Zelle 13. Mit anderen Worten befindet sich der Verbindungssteg 15 vollständig in der Fläche der Zelle 13, die von den beiden Stegpaare 14a, 14b begrenzt ist. Aus Gründen der Übersichtlichkeit ist die Fläche der betreffenden Zelle 13 grau hinterlegt.

Der Verbindungssteg 15 unterteilt die Zelle 13 in zwei Unter- oder Teilzellen, insbesondere in zwei Halbzellen, konkret zwei im Wesentlichen symmetrische Halbzellen, die zwischen den Stegen 14a, 14b und dem Verbindungssteg 15 angeordnet sind. Jede Teilzelle bildet, für sich genommen, ebenfalls eine geschlossene Zelle. Die geteilte Zelle 13 bildet dabei die Hauptzelle, die das Design der Gitterstruktur 13 bestimmt. Die Hauptzelle ist rautenförmig und weist an den Rautenspitzen die X-förmigen Stegverbinder 12 auf. Konkret sind vier X-förmige Stegverbinder 12 pro Hauptzelle vorgesehen.

In Fig. 1 ist gut zu erkennen, dass die Zellen 13 eines ersten Umfangssegment 16 (grau) alle entsprechend aufgebaut sind und jeweils einen Verbindungssteg 15 aufweisen. In Fig. 1 ist weiter zu erkennen, dass die in Axialrichtung angrenzenden Umfangssegmente 17 entsprechend dem ersten Umfangssegment 16 ausgebildet sind, sodass sich ein in Axialrichtung erstreckender Bereich aus Zellen 13 ergibt, der die gleichen Konstruktionsmerkmale und Funktionen bzw. Eigenschaften hat. Die Randzellen sind ebenfalls rautenförmig, aber ohne Verbindungsstege 15 aufgebaut. Andere Konfigurationen der Gitterstruktur 10 sind möglich.

Der Verbindungssteg 15 ist gekrümmt, insbesondere S-förmig gekrümmt. Andere Formen des Verbindungssteges 15 sind möglich.

In Fig. 2 ist gut zu erkennen, dass der Verbindungssteg 15 die Zelle 13 im Wesentlichen diagonal überbrückt. Die diagonale Anordnung des Verbindungssteges 15 bedeutet, dass die Lage des Verbindungssteges 15 zumindest von einer der beiden Achsen, insbesondere von beiden Achsen der Zelle 13 abweicht, konkret diese schneidet bzw. kreuzt. Die Achsen der Zelle 13 sind in Fig. 2 nicht eingezeichnet und umfassen die Längsachse und die Hochachse der Zelle 13. Die Längsachse erstreckt sich zwischen den beiden weiter entfernt angeordneten Stegverbindern 12 der Zelle 13. Die Hochachse ist kürzer als die Längsachse. Dies gilt jedenfalls für eine rautenförmige Zelle 13. Die Längsachse der Zelle 13 verläuft im Wesentlichen parallel zur Stentachse. Im aufgedehnten Zustand der Zelle 13 kann sich die Zellform allerdings so ändern, dass die Zelle 13 höher als breit ist, ebenso wie sie sich je nach Aufdehnzustand etwas dreht, so dass man nicht streng "parallel zur Stentachse" sagen kann.

Mit dem Begriff diagonal ist keine streng geometrische Diagonalität gemeint.

Durch die diagonale Anordnung des Verbindungssteges 15 wird erreicht, dass der Verbindungssteg 15 abschnittsweise, konkret im mittleren Bereich des Verbindungssteges 15, in etwa parallel zu den Stegen 11 des zweiten Stegpaares 14b verläuft. Dies gilt zumindest für den expandierten Zustand. Der S-förmige Verbindungssteg 15 ist so angeordnet, dass sich dieser dem jeweiligen Steg 11 des ersten Stegpaares 14a im Wesentlichen tangential annähert.

Bei dem Ausführungsbeispiel sind die Stege 11 des zweiten Stegpaares 14b gerade. Der gekrümmte Verbindungssteg 15 erstreckt sich im Wesentlichen in derselben Richtung wie die geraden Stege 11 des zweiten Stegpaares 14b.

Die relative Anordnung des Verbindungssteges 15 in der Zelle 13 kann sich in Abhängigkeit vom Kompressions- bzw. Expansionszustand der Gitterstruktur 10 ändern.

Fig. 1, 2 zeigt die Abwicklung der Gitterstruktur 10, also das Schnittmuster, so wie die Struktur geschnitten wird. Die röhrchenförmige Gitterstruktur 10 wird aufgedehnt, so dass die Zellen 13 sich verkürzen und erhöhen. Der Zellwinkel, also der Winkel zwischen den Stegpaaren 14a und 14b, nimmt dabei zu. Im voll expandierten Zustand, also wenn man die Gitterstruktur 10 ins Freie entlässt, beträgt der Zellwinkel etwa zwischen 100 und 110°, im Gefäß zwischen 20° bis 30° am unteren zulässigen Einsatzdurchmesser und 90° am oberen Einsatzdurchmesser. Andere Zellwinkel sind möglich.

Die Stegbreite des Verbindungssteges 15 variiert. Wie in Fig. 2 zu erkennen, ist die Stegbreite im Bereich der axialen Enden des Verbindungssteges 15 kleiner als die Stegbreite des Verbindungssteges 15 im mittleren Bereich, also in dem Bereich der sich zwischen den beiden Enden des Verbindungssteges 15 erstreckt. Die Stegbreite im Bereich der Enden ist mit S4 und im mittleren Bereich mit S3 bezeichnet. Dadurch wird einerseits die Stützkraft des Verbindungssteges 15 im Bereich der Stegbreite S3 erhöht und andererseits die Biegeflexibilität im Bereich der Anbindung (Stegbreite S4) verbessert.

Die Anbindung des Verbindungssteges 15 erfolgt durch einen Z-förmigen Stegverbinder 18. Die Z-Form des Stegverbinders 18 bedeutet, dass das Ende des einen Verbindungssteges 15 und das Ende des nächsten Verbindungssteges 15 der angrenzenden Zelle zusammen mit dem Steg 11, mit dem die beiden Stegverbinder 15 verbunden sind, annähernd ein Z bilden. Durch die Krümmung, insbesondere S-förmige Krümmung des Verbindungssteges 15 ergibt sich eine annähernd tangentiale Annäherung des Endes des Verbindungssteges 15 an den Steg 11 im Bereich des Z-förmige Stegverbinders 18.

Mit anderen Worten sind die beiden waagrechten Schenkel des Z gekrümmt und nähern sich in etwa tangential dem Steg 11 an. In diesem verjüngt sich die Stegbreite ausgehend von S3 im mittleren Bereich auf S4 im Endbereich des Verbindungssteges (Taper). Sie nimmt dann zum Verbinder hin wieder zu. Durch die Lage, Länge und Stärke der Stegbreitenreduktion (Taper) kann man sowohl die Crimpbarkeit als auch die Wandapposition optimieren.

Die Stegbreite S1 des Steges 11 ist größer als die Stegbreite S4 des Verbindungssteges 15 im Bereich des Stegverbinders 18. Dies ist vorteilhaft, aber nicht zwingend.

Der Verbindungssteg des Stegverbinders 18 zwischen den beiden angrenzenden Verbindungsstegen 15 hat im wesentlichen dieselbe Stegbreite S 1 wie der übrige Steg 11 des ersten Stegpaares 14a, in den die Verbindungsstege 15 einmünden.

Durch den Z-förmigen Stegverbinder 18 wird erreicht, dass die Verbindungsstege 15 der beiden benachbarten Zellen 13 nicht miteinander fluchten. Vielmehr sind deren Verbindungsstellen mit der Zelle 13 so zueinander versetzt, dass sich im komprimierten Zustand Abschnitte der unmittelbar benachbarten Verbindungsstege 15 überlappen. Dadurch wird die Crimpbarkeit der Vorrichtung bzw. des Stents verbessert, sodass im komprimierten Zustand kleine Durchmesser der Gitterstruktur 10 erzielt werden können.

Außerdem trägt die Z-Form des Stegverbinders 18 zur Biegeflexibilität der Gitterstruktur 10 bei. Z-förmige Stegverbinder 18 weisen eine größere Flexibilität als X-förmige Stegverbinder 12 auf. Die flexible Anbindung des Verbindungsstegs 15 durch den Stegverbinder 18 ist besonders vorteilhaft. Der Vorteil wird durch die verjüngten Enden der Verbindungsstege 15 noch weiter verbessert.

Die Überlappung der Verbindungsstege 15 im komprimierten Zustand kann durch den Versatz zwischen den Einmündungsstellen bzw. Verbindungsstellen der Verbindungsstege 15 und des Steges 11 des ersten Stegpaares 14a eingestellt werden. Der Versatz zwischen den Einmündungs- bzw. Verbindungsstellen der Verbindungsstege 15 (also die Länge der Überlappung) kann zwischen dem 0,1- und dem 0,9-fachen der Steglänge des Steges 11 des ersten Stegpaares 14a der Zelle 13 betragen, insbesondere zwischen dem 0,2- und 0,8-fachen, insbesondere zwischen dem 0,3- und 0,7-fachen.

Die im Zusammenhang mit einem Verbindungssteg 15 beschriebenen Kontruktionsmerkmale und Eigenschaften werden auch im Zusammenhang mit den entsprechend ausgebildeten Verbindungsstegen 15 der anderen Zellen 13 offenbart und beansprucht.

Die Verstärkung der Zelle durch den Verbindungssteg 15 ermöglicht eine Modifikation der Gitterstruktur 10 dahingehend, dass die Biegeflexibilität erhöht werden kann, ohne dass dabei die Radialkraft bzw. Stützkraft der Gitterstruktur 10 im implantierten Zustand beeinträchtigt wird. Vielmehr kann durch entsprechende Auslegung der Verbindungsstege 15 die Stützkraft bei erhöhter Biegeflexibilität gleichmäßig auf die Gefäßwand verteilt werden.

Die Erhöhung der Biegeflexibilität kann beispielsweise dadurch erreicht werden, dass die Anzahl der Zellen 13 pro Umfangssegment 16 verringert wird. Dies führt zu einer Konfiguration der Gitterstruktur 10, die, wie in Fig. 1 gezeigt, wenigstens einen Abschnitt, insbesondere im mittleren Bereich der Vorrichtung mit einer geringen Anzahl von Zellen 13, insbesondere 3 Zellen 13 pro Umfangssegment 16 aufweist. Ein weiterer Abschnitt, insbesondere die Randbereiche der Vorrichtung weist mehr Zellen 13, insbesondere 6 Zellen pro Umfangssegment auf. Allgemein gesagt kann mit der Erfindung die Anzahl der Zellen 13 pro Umfangssegment 16 verringert werden.

Die Funktion der diagonalen Stege besteht darin, die durch diese Reduktion der Anzahl der Zellen verlorengehende Stützwirkung in den Zwischenräumen auszugleichen, ohne dabei aber die Biegeflexibilität zu beeinträchtigen.

Bei dem Ausgangsbeispiel gemäß Fig. 1 sind 3 Zellen pro Umfangssegment im mittleren Bereich der Vorrichtung vorgesehen. Eine andere Anzahl von Zellen ist möglich, wobei aber für die Biegeflexibilität gilt, dass diese abnimmt, je mehr Zellen pro Umfangssegment vorgesehen sind.

Fig. 3 zeigt ein weiteres erfindungsgemäßes Ausführungsbeispiel, wobei 2 Zellen 13 pro Umfangssegment 16 vorgesehen sind. Es ist zu erkennen, dass die Zellen 13 eines Umfangssegment 16 jeweils zwei Verbindungsstege 15 aufweisen, die die Zellen 13 jeweils im Wesentlichen diagonal überbrücken. Die Anbindung der Verbindungsstege 15 erfolgt durch Z-förmige Stegverbinder 18. Die Verbindungsstege 15 unterteilen die Zelle 13 in drei Unter- oder Teilzellen, die zwischen den Stegen 14a, 14b und den Verbindungsstegen 15 angeordnet sind. Jede Teilzelle bildet, für sich genommen, ebenfalls eine geschlossene Zelle. Die geteilte Zelle 13 bildet dabei die Hauptzelle, die das Design der Gitterstruktur 10 bestimmt. Bei diesem konkreten Ausführungsbeispiel sind die Stegverbinder 12 des ersten Umfangssegments 16 im expandierten Zustand der Gitterstruktur 10 auf dem Umfang um einen Winkel von 180° versetzt. Die Form der Hauptzelle gemäß Fig. 3 entspricht der Form der der Hauptzelle gemäß Fig. 2. Deshalb wird auf die obigen Ausführungen verwiesen.

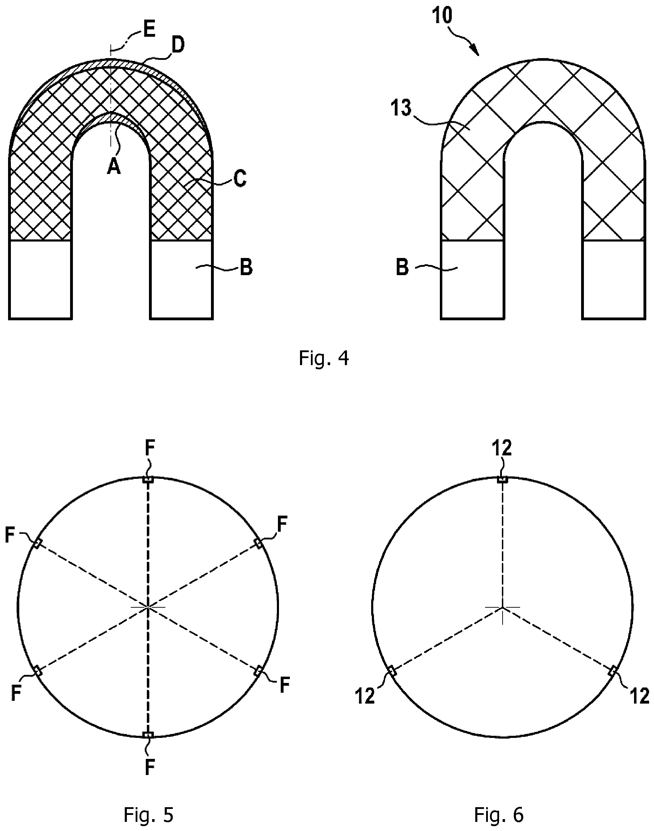

Die Wirkung des erfindungsgemäßen Ausführungsbeispiels gemäß der Figuren 1 und 2 ist in Fig. 4 dargestellt.

In der linken Darstellung der Fig. 4 ist ein engmaschiger Stent C mit einer 6-Zellen-Struktur, d.h. sechs Zellen pro Umfangssegment, als Vergleichsbeispiel schematisiert gezeigt. Der Stent C ist in einem standardisierten U-Bogen B als Gefäßmodell angeordnet. Unterhalb eines bestimmten Krümmungsradius (Radius of Curvature) der Innenkurve A hebt sich die Stentstruktur von der Außenkurve D des Bogens B ab, weil sich die Stentzellen nicht mehr weiter strecken können. An der Innenkurve A kommt es zu einem Einknicken des Stents C, weil sich die Zellen nicht mehr weiter stauchen lassen.

Die Wandapposition, also das Anliegen des Closed-Cell-Stents C an der Gefäßwand, nimmt dabei sowohl auf der Innen- als auch auf der Außenseite des Gefä-ßes mit zunehmender Krümmung ab. Die Krümmung ist dabei der Kehrwert des Radius der Mittellinie. Auf der Außenseite werden die rautenförmigen Zellen mehr und mehr in axialer Richtung gestreckt, bis sie sich nicht mehr weiter strecken können und von der Gefäßwand abheben. Auf der Innenseite kommt es hingegen zunächst zu einer Stauchung der Rauten und dann schließlich zu einem Kollabieren der Struktur ins Innere des Gefäßlumens.

In der rechten Darstellung der Fig. 4 ist ein Stent nach einem erfindungsgemäßen Ausführungsbeispiel mit einer 3-Zellen-Struktur schematisiert gezeigt. Die Effekte in der linken Darstellung treten im U-Bogen B mit dem gleichen Krümmungsradius nicht oder in geringerem Maße auf. Dies liegt an der verbesserten Biegeflexibilität der Gitterstruktur 10 aufgrund der vergleichsweise geringeren Anzahl von Zellen 13 pro Umfangssegment. Die geringere Anzahl von Zellen 13 pro Umfangssegment ist durch die Verbindungsstege 15 (in Fig. 4 nicht dargestellt) möglich, die in den betreffenden Zellen 13 angeordnet sind und die Radialkraft der Gitterstruktur 10 erhöhen. Die mit der geringen Anzahl von Zellen 13 pro Umfangssegment einhergehende niedrige Stützwirkung wird durch die Verbindungsstege 15 kompensiert und kann sogar weiter erhöht werden.

Fig. 5 zeigt den Querschnitt E aus Fig. 4, linke Darstellung, also die Abbildung der Stege einer 6-Zellen-Struktur als Vergleichsbeispiel. Dem ist Fig. 6 gegenüber gestellt, die einen entsprechenden Querschnitt durch den Stent C aus Fig. 4, rechte Darstellung zeigt, also die Abbildung der Stege einer 3-Zellen-Struktur als erfindungsgemäßes Ausführungsbeispiel.

Das 3-Zellen-Design hat bei einer Biegung um eine quer zur Längsachse des Stents liegende Achse eine bessere Biegeflexibilität als ein 6-Zellen-Design mit entsprechenden Dimensionen. Dies lässt sich am besten sehen, wenn man die Stegquerschnitte in einer zur Längsachse orthogonalen Ebene betrachtet, die in einem Testaufbau (U-Bogen) an der Stelle der maximalen Krümmung liegt, vgl. Figuren 5, 6.

Bei dem 6-Zellen-Design liegt jedem Verbinder F genau ein weiterer Verbinder F diametral gegenüber, vgl. Fig. 5. Das bedeutet bei der Biegung um eine Achse quer zur Längsachse des Stents, dass einem Verbinder F, der auf der Außenlinie der Biegung liegt und maximal auf Zug belastet wird, auf der Innenlinie der Biegung auch gerade ein Verbinder F gegenüberliegt, der maximal auf Druck belastet wird. Im Ergebnis ergibt sich ein hohes Biegewiderstandsmoment gegen die Biegung.

Bei einem 3-Zellen-Design hingegen liegt bei gleicher Ausrichtung des auf der Außenlinie der Biegung liegenden Stegverbinders 12 kein Stegverbinder direkt gegenüber, sondern die beiden anderen Stegverbinder 12 sind um jeweils 120° versetzt, vgl. Fig. 6. Ein reines 3-Zellen-Design hätte aber zu große Zellöffnungen, um die Funktion des Stents für Stenose oder für Stent-Assisted Coiling zu erfüllen. Die Verbindungsstege 15 ermöglichen hingegen eine gute Stützwirkung, ohne die Biegesteifigkeit wie bei einem 6-Zellen-Design zu erhöhen.

Die Anbindung der Verbindungsstege 15 an die durchlaufenden Stege 11 der 3-Zellen-Struktur erfolgt vorteilhaft über Z-förmige Stegverbinder 18, die insbesondere auf der Außenlinie der Biegung eine weitere Dehnung der Gitterstruktur 10 zulassen als wenn die neutralen Linien benachbarter diagonaler Verbindungsstege 15 direkt fluchten würden. Die Stegbreite der Verbindungsstege 15 kann in der Nähe der Z-förmigen Stegverbinder 18 lokal reduziert werden, um eine für die Anwendung optimale Verformung der Verbindungsstege 15 zu erzielen. Insbesondere kann dadurch die Wandapposition der Verbindungsstege 15 an der Innenkurve der Biegung nochmals verbessert werden.

Da die Zellenstruktur nach dem erfindungsgemäßen Ausführungsbeispiel zwar breitere, aber dafür weniger Stege 11 aufweist als eine entsprechende 6-Zellen-Struktur mit gleicher Radialkraft, sind vergleichbare Stents durch das gleiche oder sogar ein kleineres Katheterlumen zuführbar.

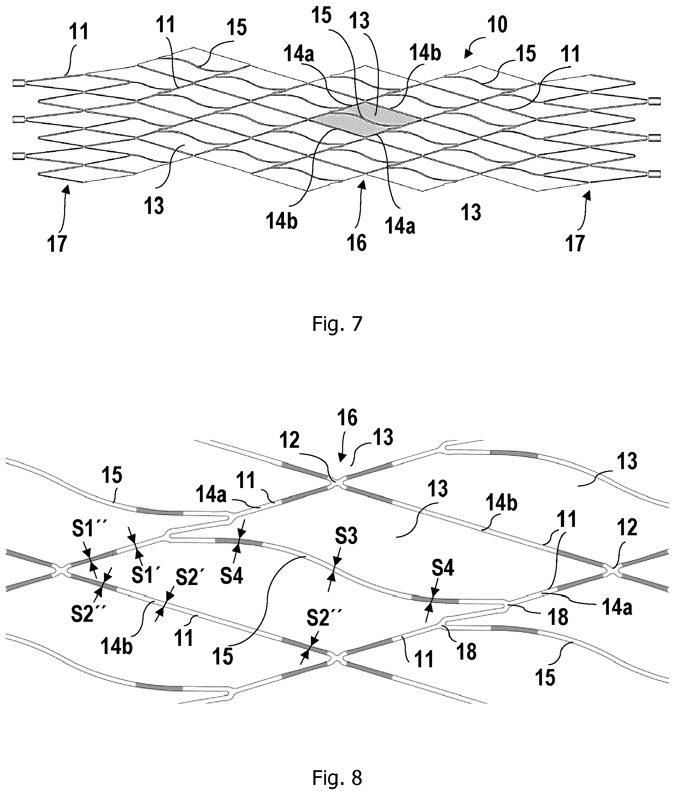

Fig. 7 zeigt ein weiteres erfindungsgemäßes Ausführungsbeispiel, wobei 3 Zellen 13 pro Umfangssegment 16 vorgesehen sind. Die Zellen 13 eines Umfangssegment 16 weisen jeweils einen Verbindungssteg 15 auf, der die Zellen 13 jeweils im Wesentlichen diagonal überbrückt. Es ist weiter zu erkennen, dass die Zellen 13 der in Axialrichtung angrenzenden Umfangssegmente (Randsegmente) 17 ebenfalls rautenförmig, aber ohne Verbindungsstege 15 aufgebaut sind. Es ist denkbar, dass die Zellen 13 der Randsegmente 17 des proximalen Endes der Gitterstruktur 10 und die Zellen 13 der Randsegmente 17 des distalen Endes unterschiedlich ausgebildet sind (nicht dargestellt). Auf diese Weise kann erreicht werden, dass die Zellen 13 des proximalen und distalen Endes der Gitterstruktur 10 unterschiedliche Eigenschaften aufweisen. So kann das Flaring beispielsweise am proximalen Ende der Gitterstruktur 10 stärker ausgebildet sein als am distalen Ende der Gitterstruktur 10.

Die Stegbreite der Stege 11 des ersten und zweiten Stegpaares 14a, 14b variiert. Wie in Fig. 8 zu erkennen, ist die Stegbreite der Stege 11 in einem Bereich nahe der X-förmigen Stegverbinder kleiner ausgebildet als die Stegbreite der Stege 11 in einem Bereich nahe der Z-förmigen Stegverbinder. Die Stegbreite der Stege 11 des ersten Stegpaares 14a im Bereich nahe der X-förmigen Stegverbinder ist mit S1" und im Bereich nahe der Z-förmigen Stegverbinder mit S1' bezeichnet. Die Stegbreite der Stege des zweiten Stegpaares 14b im nahe Bereich der X-förmigen Stegverbinder ist mit S2" und im Bereich nahe der Z-förmigen Stegverbinder mit S2' bezeichnet. Dadurch wird einerseits die Stützkraft der Stege 11 im Bereich der Stegbreiten S1', S2' erhöht und andererseits die Biegeflexibilität im Bereich der Anbindung bzw. der X-förmigen Stegverbinder (Stegbreite S1", S2") verbessert.

Das Verhältnis der Stegbreite S1' zur Stegbreite S1" sowie das Verhältnis der Stegbreite S2' zur Stegbreite S2" beträgt zwischen 0,5 und 1,0, wie beispielswiese ca. 0,75. Das Verhältnis der Stegbreite S3 zur Stegbreite S4 des Verbindungsstegs 15 beträgt zwischen 0,7 und 1,0, beispielsweise 0,95.

Die im Zusammenhang mit einem 3-Zellen-Design beschriebenen Vorteile und Konstruktionsmerkmale lassen sich im Prinzip auch mit einer Gitterstruktur realisieren, die eine andere Anzahl als 3 Zellen pro Umfangssegment aufweist.

Das Zellendesign eignet sich prinzipiell für alle klinischen Anwendungen, bei denen eine Gefäßanatomie mit einem Dauerimplantat oder auch einem temporär eingesetzten Implantat mit möglichst guter Wandapposition behandelt werden soll, ohne dass es zu einem Kollabieren der Struktur kommt. Diese meist selbstexpandierenden Implantate werden vorzugsweise aus Nitinolrohr lasergeschnitten, mit Wärmebehandlung auf ihren Enddurchmesser gebracht und elektropoliert. Andere Legierungen oder Herstellungsverfahren sind denkbar.

Zusammengefasst ermöglicht die Erfindung bzw. das vorstehend beschriebene Ausführungsbeispiel ein Zellendesign für lasergeschnittene Stents mit geschlossenen Zellen (Closed-Cell-Design), das ein Einschnüren und Einknicken der Struktur in stärker gekrümmten Gefäßen reduziert, das bei einem Closed-Cell-Design nach dem Stand der Technik ab einer bestimmten Krümmung auftritt. Dabei werden die positiven Eigenschaften des Designs, Crimpbarkeit auf ein enges Katheterlumen, Wiedereinziehbarkeit, engmaschige Abdeckung der Gefäßwand weitgehend erhalten (Abdeckung von Aneurysmenhälsen), komplett erhalten (Wiedereinziebarkeit/Resheathability) oder gar verbessert (Radialkraft, Crimpbarkeit auf ein enges Katheterlumen).

### Bezugszeichenliste

- 10: Gitterstruktur
- 11: Stege
- 11a: erster Steg
- 11b: zweiter Steg
- 12: erste Stegverbinder
- 13: Zellen
- 14a: erstes Stegpaar
- 14b: zweites Stegpaar
- 15: Verbindungssteg
- 16: erstes Umfangssegment
- 17: zweites Umfangssegment
- 18: zweite Stegverbinder (Z-förmig)

## Patentansprüche

1. Medizinische Vorrichtung zur Einfuhr in ein Körperorgan mit einer komprimierbaren und expandierbaren Gitterstruktur (10) aus Stegen (11), die durch Stegverbinder (12) miteinander verbunden sind und geschlossene Zellen (13) der Gitterstruktur (10) begrenzen, wobei jeweils zwei Stege (11a, 11b) wenigstens einer Zelle (13) gegenüber angeordnet sind und ein erstes Stegpaar (14a) und ein zweites Stegpaar (14b) bilden,
**dadurch gekennzeichnet, dass**
eines der beiden Stegpaare (14a, 14b) durch mindestens einen Verbindungssteg (15) miteinander verbunden ist, der sich in die Zelle (13) erstreckt und diese überbrückt.

2. Medizinische Vorrichtung nach Anspruch 1
**dadurch gekennzeichnet, dass**
die Zelle (13) im Wesentlichen rautenförmig ist.

3. Medizinische Vorrichtung nach Anspruch 1 oder 2
**dadurch gekennzeichnet, dass**
mehrere Zellen (13) auf wenigstens einem, insbesondere wenigstens einem ersten Umfangssegment (16) angeordnet sind, bei denen jeweils eines der beiden Stegpaare (14a, 14b) durch mindestens einen Verbindungssteg (15) miteinander verbunden ist, der sich in die Zelle (13) erstreckt und diese überbrückt.

4. Medizinische Vorrichtung nach Anspruch 3
**dadurch gekennzeichnet, dass**
die Stegverbinder (12) des ersten Umfangssegments (16) auf dem Umfang um einen Winkel versetzt zueinander angeordnet sind, der kleiner oder gleich 180° ist.

5. Medizinische Vorrichtung nach einem der Ansprüche 1 bis 4
**dadurch gekennzeichnet, dass**
der Verbindungssteg (15) die Zelle (13) diagonal überbrückt.

6. Medizinische Vorrichtung nach einem der Ansprüche 1 bis 5
**dadurch gekennzeichnet, dass**
der Verbindungssteg (15) mit dem Stegpaar (14a) jeweils durch einen flexiblen, insbesondere Z-förmigen Stegverbinder (18) verbunden ist.

7. Medizinische Vorrichtung nach einem der Ansprüche 1 bis 6
**dadurch gekennzeichnet, dass**
die Stegbreite des Verbindungsstegs (15) im Bereich seiner Enden kleiner als die Stegbreite in einem Bereich ist, der sich zwischen den Enden erstreckt.

8. Medizinische Vorrichtung nach einem der Ansprüche 1 bis 7
**dadurch gekennzeichnet, dass**
der Verbindungssteg (15) einer ersten Zelle (13) und der Verbindungssteg (15) einer in Umfangsrichtung benachbarten zweiten Zelle (13) versetzt zueinander angeordnet sind.

9. Medizinische Vorrichtung nach einem der Ansprüche 3 bis 8
**dadurch gekennzeichnet, dass**
das Umfangssegment (16) wenigstens zwei Zellen (13) aufweist.

10. Medizinische Vorrichtung nach einem der Ansprüche 3 bis 9
**dadurch gekennzeichnet, dass**
die Anzahl der Zellen (13) auf dem ersten Umfangssegment (16) geringer ist als die Anzahl der Zellen (13) auf wenigstens einem zweiten Umfangssegment (17), das in axialer Richtung der Gitterstruktur (10) proximal und/oder distal entfernt vom ersten Umfangssegment (16) angeordnet ist.

11. Behandlungssystem mit einer medizinischen Vorrichtung nach einem der Ansprüche 1 bis 10 und mit einem Katheter, in dem ein Führungselement mit einem axialen Ende der Gitterstruktur (10) verbunden ist.
